# EUROPEAN PATENT APPLICATION

(11) **EP 2 281 804 A1**
(43) Date of publication of application: **09.02.2011**
(21) Application number: 09176514.9
(22) Date of filing: 19.11.2009
(51) Int. Cl.: C07C 231/24, C07C 237/46

(54) **A continuous process of preparing 5-amino-N,N'-bis(2,3-dihydroxypropyl)-2,4,6-triiodoisophthalamide, an intermediate for synthesizing non-ionic X-ray contrast agents**

(30) Priority: 21.07.2009 US 227085 P
(71) Applicant: GE Healthcare AS, 0401 Oslo (NO)
(72) Inventor: Askildsen, Arne, 4521 Spangereid (NO); Holmaas, Lars, Terje, 4521 Spangereid (NO); Sørensen, Thore, Jarle, 4517 Mandal (NO)
(74) Representative: Wulff, Marianne Weiby

(57) **Abstract**

This invention relates to an improved method for the synthesis of 5-amino-N,N'- bis(2,3-dihydroxypropyl)-2,4,6-triiodoisophthalamide (Compound B), an intermediate in the industrial preparation of non-ionic X-ray contrast agents. In particular, it relates to a continuous process of the iodination reaction of 5-amino-N,N'-bis(2,3-dihydroxypropyl-isophthalamide (ABA) or ABA-HCl followed by the purification of Compound B.

## Description

### TECHNICAL FIELD

This invention relates generally to large-scale synthesis of non-ionic X-ray contrast agents. It further relates to an improved method for the synthesis and purification of 5-amino-N,N'-bis(2,3-dihydroxypropyl)-2,4,6-triiodoisophthalamide (Compound B), a key intermediate in the industrial preparation of non-ionic X-ray contrast agents. In particular, it relates to a continuous process for the iodination reaction and the subsequent purification of Compound B.

### BACKGROUND OF THE INVENTION

Non-ionic X-ray contrast agents constitute a very important class of pharmaceutical compounds produced in large quantities. 5-[N-(2,3-dihydroxypropyl)-acetamido]-N,N'-bis(2,3-dihydroxypropyl)-2,4,6-triiodo-isophthalamide ("iohexol"), 5-[N-(2-hydroxy-3-methoxypropyl)acetamido]-N,N'-bis(2,3-dihydroxypropyl)-2,4,6-triiodo-isophthalamide ("iopentol"), N,N'-Bis(2,3-dihydroxypropyl)-5-[(2-hydroxyacetyl)-(2-hydroxyethyl)amino]-2,4,6-triiodo-benzene-1,3-dicarboxamide ("ioversol"), and 1,3-bis(acetamido)-N,N'-bis[3,5-bis(2,3-dihydroxypropyl-aminocarbonyl)-2,4,6-triiodophenyl]-2-hydroxypropane ("iodixanol") are important examples of such compounds. They generally contain one or two triiodinated benzene rings.

For example, iodixanol, marketed under the trade name Visipaque®, is one of the most used agents in diagnostic X-ray procedures. It is produced in large quantities by GE Healthcare in Lindesnes, Norway. The industrial production of iodixanol involves a multistep chemical synthesis as shown in Scheme 1 below. *See also* U.S. Patent Nos. 6,274,762 and RE38,856. To reduce the cost of the final product, it is critical to optimize each synthetic step. Even a small improvement in reaction design can lead to significant savings in a large scale production.

In the iodination reaction, 5-amino-N,N'-bis(2,3-dihydroxypropyl)-isophthalamide (also known as aminoisophthalic acid bisamide, AIPA bisamid or ABA) is iodinated by iodine chloride to produce Compound B. The instant improvement is directed to a continuous iodination and purification process for preparing Compound B.

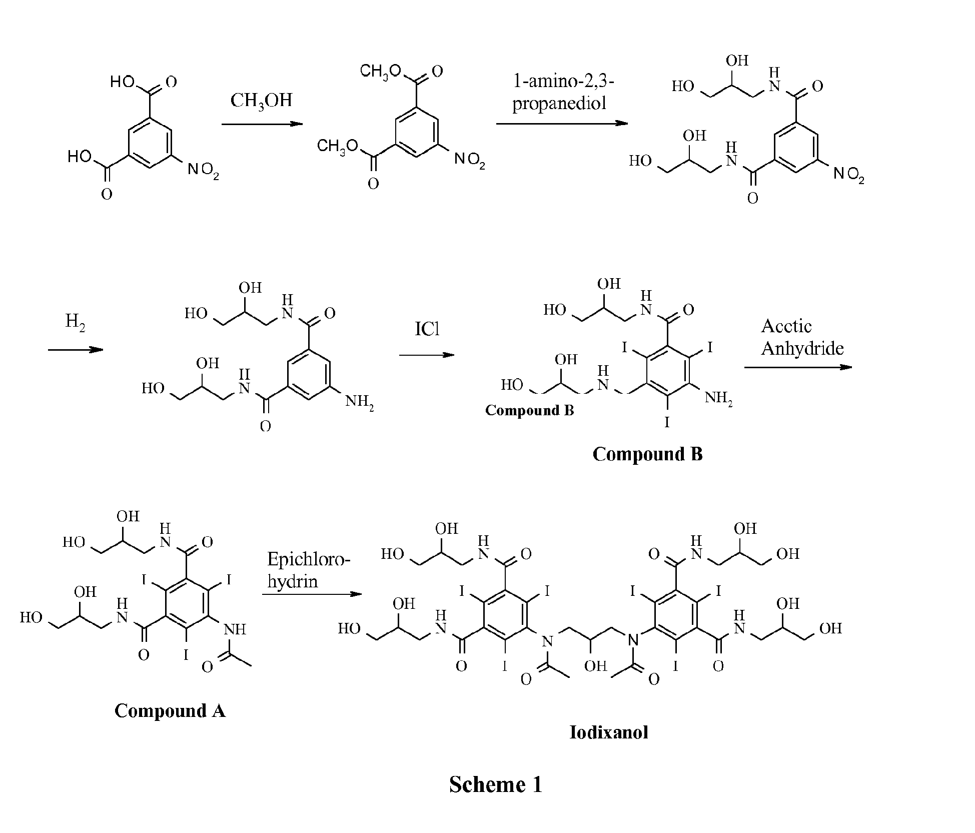

### BRIEF DESCRIPTION OF THE DRAWINGS

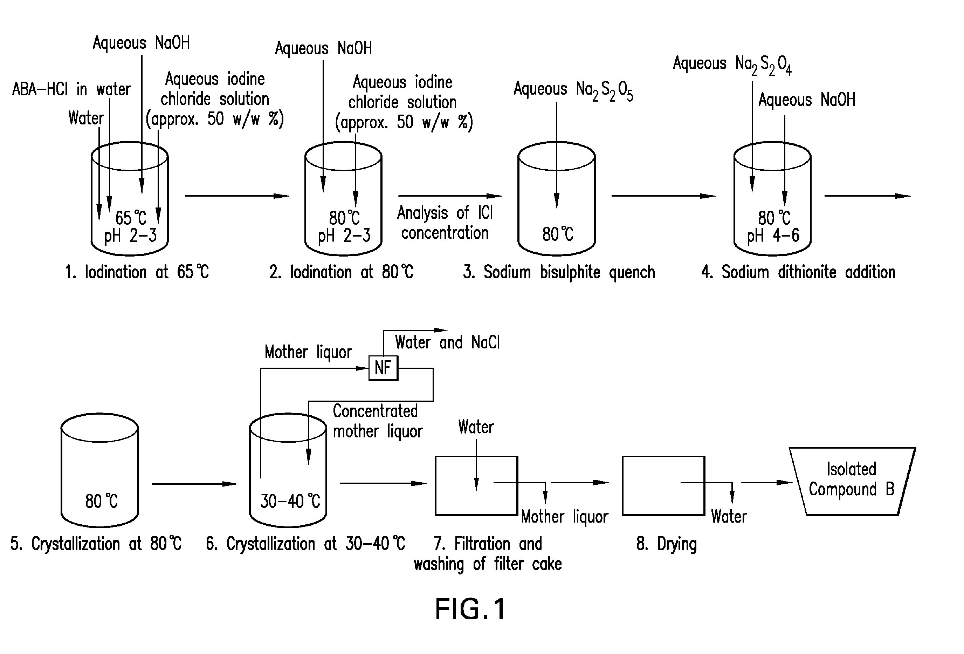

FIG. 1 is a schematic diagram of one exemplary embodiment of the instant invention.

### SUMMARY OF THE INVENTION

The present invention provides an industrial process for preparing and purifying Compound B. Specifically, it uses a continuous process comprising the steps of (1) iodinating 5-amino-N,N'-bis(2,3-dihydroxypropyl)-isophthalamide (ABA) or ABA-HCl using iodine chloride at a pH between about 2 and about 3 and at a temperature between about 60 °C and about 90 °C in a first reactor; (2) quenching the iodination reaction in a second reactor by adding a quenching reagent; (3) decolourising the quenched reaction mixture in a third reactor at a pH above about 4 by adding a decolourising reagent; (4) crystallizing the decolourised mixture by cooling to a temperature of about 25 °C and about 45 °C in a fourth reaction; and (5) filtering and washing the crystals of Compound B.

A reactor in the instant process may be a tubular flow reactor, a continuous stirred tank reactor (CSTR), or other suitable continuous reaction equipment.

### DETAILED DESCRIPTION OF THE INVENTION

Compound B can be made by iodination of ABA or its hydrochloride form, ABA-HCl, as shown in Scheme 2 below:

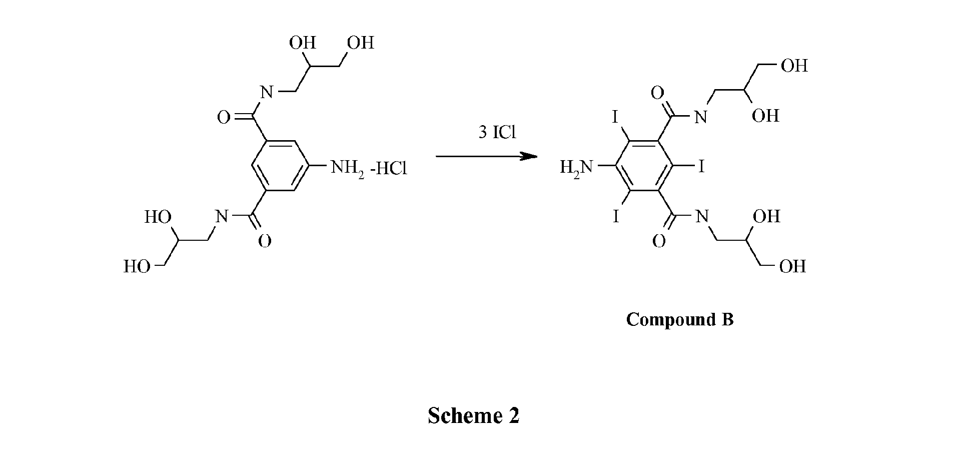

Traditionally, the iodination reaction is run in batch mode. In a batch process, all the operations are performed successively in the same reactor. Thus, production capacity increase in a batch production requires very large reactor volumes and a corresponding increase in capital investments. On the other hand, a continuous process has dedicated equipment for every operation, where the mixture moves from one operation to the next within the production line. All the operations are performed continuously all the time and the system is at a steady state. Consequently, a continuous production process requires much smaller equipment volumes and investments for achieving the same production capacity. In addition, a continuous operation allows for less varying quality of the product. While desirable, a continuous system is more complicated to design and highly specific to one single product.

We have however found that the iodination reaction and the subsequent purification of Compound B can be performed in a continuous mode. The instant continuous process gives significant benefits in reduced cycle times, reduced amount of equipment required in a production line, reduced headcount and a stabilized process with resulting consistent product quality in terms of yield, impurity profile and physical characteristics of the product.

The continuous process of the present invention is carried out in several steps. First, Compound B is produced in a reactor by tri-iodination of ABA or ABA-HCl at pH about 2 to about 3 at a temperature of between about 60 °C and about 90 °C. The concentration of ABA or ABA-HCl in the iodination reaction step can be varied, but will typically be in the range of 15-40 w/v %. Higher dilutions are also possible, but will be less favorable with regard to process efficiency. The pH in the iodination step is kept below about 4 to avoid degradation of iodine chloride. The pH is also kept above about 1 to avoid protonation of the amino group of ABA, which may deactivate the iodination reaction. Protonation of the amino group is however a reversible reaction.

In a preferred embodiment, the reactor for the iodination reaction is a CSTR. ABA (or ABA-HCl) is dissolved or suspended in water before introduction to the CSTR. ABA-HCl or ABA, water, aqueous iodine chloride solution and aqueous sodium hydroxide are continuously added to the CSTR. Throughout the iodination reaction, continuous addition of small amounts of aqueous sodium hydroxide may become necessary to maintain the pH at about 2 to about 3. In addition, minor amounts of aqueous iodine chloride solution may be added continuously to maintain the conversion to Compound B at about or higher than 98 %. The iodination reaction may preferably begin at a relatively low temperature, e.g., at about 65°C. Further iodination may be performed at a higher temperature, e.g., at about 80°C, preferably in a second CSTR. The additional iodination at a higher temperature may facilitate the addition of all three iodine atoms to the benzene ring.

Second, the iodination reaction is quenched in a second reactor using a quenching reagent. Preferably, the excess iodine chloride is quenched in a CSTR where an aqueous solution of sodium bisulphite (Na₂S₂O₅) is added simultaneously to the feed from the last iodination reactor. The flow of sodium bisulphite solution may optionally be adjusted according to an on-line measurement of the iodine chloride concentration in the feed, e.g., by Raman spectroscopy or other spectroscopical techniques. The process solution coming out of the quenching reactor is devoid of iodine chloride.

Third, the quenched reaction mixture is decolourised in a third reactor at a pH above about 4 by adding a decolourising reagent. Preferably, sodium dithionite (Na₂S₂O₄) is added at about 80°C and at a pH about 4 to about 6 in a CSTR. Aqueous sodium hydroxide and sodium dithionite may also be added continuously.

Fourth, crystallization of Compound B occurs in a fourth reaction by cooling the decolourised mixture to a temperature of about 25 °C and about 45 °C. Preferably, the crystallization step is carried out at about 80°C in a CSTR. Unlike a batch process, no seeding is necessary in the instant continuous process because there will always be crystals in the reactor. Further crystallization in another CSTR may be preferred, for example, at a temperature of between about 30 °C to about 40°C. The mother liquor may optionally be continuously decanted off and subjected to concentration by nanofiltration before being recycled to the CSTR. Up to about 40 % of the mother liquor volume may be removed by nanofiltration. Substantial amounts of salts (mainly sodium chloride) may be removed along with water in the nano filtration step.

Finally, crystals of Compound B are continuously filtered and washed with water or another suitable solvent. In a preferred embodiment, a continuous drying step follows continuous filtration and washing of the filter cake. These operations may be done either in separate equipment units or in combination, or in one combined unit with separate sections for each unit operation. Typical technologies for filtration include belt filtration or rotation filtration, alternatively centrifugation. Drying technologies such as belt dryers or fluid bed dryers may be employed.

The invention is illustrated further by the following examples that are not to be construed as limiting the invention in scope to the specific procedures described in them.

### EXAMPLES

### EXAMPLE 1

A slurry of ABA-HCl in water is introduced continuously to a reactor system as described in Figure 1. The initial ABA-HCl concentration in Step 1 is about 20 w/v %. About 3.1 molar equivalents of iodine chloride are used. Sodium bisulphite (0.04 molar equivalents) is used in Step 3 and sodium dithionite (0.03 molar equivalents) is used in Step 4. The temperature in Step 6 is 35 °C, and about 30 % of the mother liquor volume is removed by nanofiltration. The resulting suspension is filtered, washed with water (about L/kg product) and dried at 100-110°C in a combined filter and drying belt filter (a Pannevis filter). The resulting moisture content of the product is about 0.3-0.4 w/w % and the salt content 0.01-0.03 w/w % calculated as NaCl. The HPLC purity of Compound B is about 99 %.

All patents, journal articles, publications and other documents discussed and/or cited above are hereby incorporated by reference.

## Claims

1. A process for industrial preparation of 5-amino-N,N'-bis(2,3-dihydroxypropyl)-2,4,6-triiodoisophthalamide (Compound B) comprising the following sequential and continuous steps:
(1) iodinating 5-amino-N,N'-bis(2,3-dihydroxypropyl)-isophthalamide (ABA) or ABA-HCl using iodine chloride at a pH between about 2 and about 3 and at a temperature of between about 60 °C and about 90 °C in a first reactor;
(2) quenching the iodination reaction in a second reactor by adding a quenching reagent to a second reactor;
(3) decolourising the quenched reaction mixture in a third reactor at a pH above about 4 by adding a decolourising reagent;
(4) crystallizing the decolourised mixture by cooling to a temperature of about 25 °C and about 45 °C in a fourth reaction; and
(5) filtering and washing the crystals of Compound B.
